Europäisches Patentamt
European Patent Office
Office européen des brevets

(19)

(11) Publication number: **0 434 346 A1**

(12) # EUROPEAN PATENT APPLICATION

(21) Application number: **90313791.7**

(51) Int. Cl.[5]: **C07D 257/02**, A61K 49/04

(22) Date of filing: **17.12.90**

(30) Priority: **22.12.89 US 454890**

(43) Date of publication of application: **26.06.91 Bulletin 91/26**

(84) Designated Contracting States: **AT BE CH DE DK ES FR GB GR IT LI LU NL SE**

(71) Applicant: **E.R. SQUIBB & SONS, INC.**
**Lawrenceville-Princeton Road**
**Princeton New Jersey 08543-4000(US)**

(72) Inventor: **Dischino, Douglas, D.**
**27 Sweetgum Lane**
**Monmouth Junction, New Jersey(US)**
Inventor: **Emswiler, John**
**RD No.1 349b**
**Pittstown, New Jersey(US)**

(74) Representative: **Thomas, Roger Tamlyn et al**
**D. Young & Co. 10 Staple Inn**
**London WC1V 7RD(GB)**

(54) **10-(2'-hydroxy-3'-polyoxaalkyl)-1,4,7-triscarboxymethyl-1,4,7,10-tetraazacyclododecane.**

(57) Metal chelating ligands useful as contrast agents are disclosed having the formula

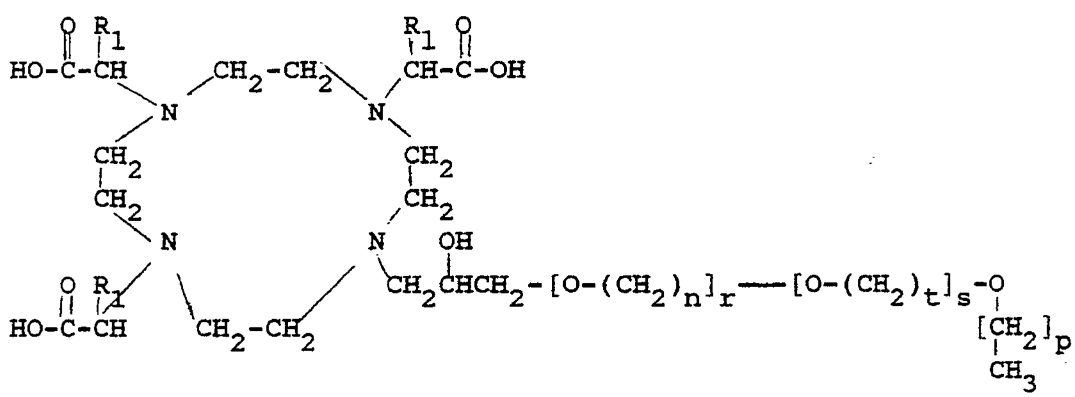

EP 0 434 346 A1

## 10-(2'-HYDROXY-3'-POLYOXAALKYL)-1,4,7-TRISCARBOXYMETHYL-1,4,7,10-TETRAAZACYCLODODECANE

Metal-chelating ligands are useful in diagnostic medicine as contrast agents. X-ray imaging, radionuclide imaging, ultrasound imaging and magnetic resonance imaging can each be enhanced by the use of a metal atom bound to a chelating ligand. For example, a chelating ligand can become a radiopharmaceutical when it is prepared as a chelate complex with $^{99m}$Tc, $^{111}$In, $^{67}$Ga, $^{140}$La, $^{169}$Yb, $^{68}$Ga, $^{90}$Y, $^{188}$Re, $^{153}$Sm or other radioactive metal ions. When a chelating ligand is complexed with the stable isotopes of the lanthanides, tantalum, bismuth or other elements with molecular weight higher than iodine, the resulting complex absorbs x-rays sufficiently to act as an x-ray contrast agent. In some cases, the agents that are useful in x-ray imaging absorb, reflect or scatter ultrasound radiation sufficiently to be used as an ultrasound agent. If a chelating ligand is complexed with a paramagnetic metal atom that has a symmetric electronic ground state (e.g., $Gd^{+3}$, octahedral ($Mn^{+2}$ or $Fe^{+3}$, $Cr^{+3}$) the resulting complex will be useful as a spin relaxation catalyst that is used in magnetic resonance imaging (also known as NMR imaging) as a contrast agent. If a chelating agent is complexed with a paramagnetic metal atom that has an unsymmetrical electronic ground state (e.g., dysprosium(III), holmium(III) and erbium(III)), the resulting complex will be useful as a chemical shift agent in magnetic resonance imaging or in magnetic resonance in vivo spectroscopy.

Desirable physical properties of the chelator differ depending on the diagnostic or therapeutic purpose of the metal chelate. Desirable physical properties common to all uses are high affinity for the metal ion bound to the chelator and ease of synthesis. when it is desired to use the metal chelate as a contrast medium for NMR imaging or general purpose X-ray imaging, the desirable physical properties are high water solubility, and viscosity and osmolality of a formulated drug as close as possible to those of human blood.

Human blood has an osmolality of 0.3 Osmol/ kg-water. Hyperosmolality is a well known contributor to adverse patient reactions to injected contrast media, and the lower osmolality of newer x-ray agents is due to their being nonionic molecules (possessing a net zero overall charge) (Shehadi, WH; "Contrast media adverse reactions: occurrence, reoccurrence and distribution patterns" Radiol. 1982, 143, 11 - 17. Bettman, MA; "Angiographic contrast agents; conventional and new media compared", Am. J. Roetgen. 1982, 139, 787 - 794. Bettman, MA and Morris TW; Recent advances in contrast agents, Radiol. Clin. North Am. 1986, 24, 347 - 357.). Gadolinium-based NMR agents in the prior art that are useful have a net negative overall charge, and therefore their aqueous formulated solutions have high osmolality. For example, $Gd(DTPA)^{2-}$ where DTPA stands for diethylenetriaminepentaacetic acid is formulated for use at 0.5 M in water as the N-methylglucamine salt. The osmolality of the solution is 1.6 to 2.0 Osmol/kg-water. The preferred new gadolinium complexes of the present invention are nonionic - they are not salts. When these nonionic gadolinium complexes are formulated at 0.5 M in water the osmolality of the solutions is 0.3 - 0.6 Osmol/kg-water. The complex should be generally inert to interaction with the body other than general tissue distribution and excretion, usually by the renal route. These properties are also important to NMR imaging, but, in addition, the effectiveness of an agent for NMR imaging can be increased by altering the chemical structure so as to increase the ability of the metal chelate to affect the relaxation times of water protons.

In radiopharmaceutical imaging the doses administered are relatively small so that matching the drug formulation's physical properties to those of human blood is relatively unimportant. In this use biological specificity is more important. In particular, one could use $^{99m}$Tc as the metal and a chelating ligand which is functionalized with a biologically active entity such as a bile acid, fatty acid, amino acid, peptide, protein, or one of numerous chemical entities known to bind receptors in vivo. NMR contrast media may also make use of biological specificity.

In radiopharmaceutical therapy, the metal ions may be chosen from among those known in the art; for example, $^{90}$Y, $^{188}$Re, $^{153}$Sm. For this purpose the chelating ligand is generally covalently bound to a disease specific entity such as a monoclonal antibody.

Using the present invention, it is possible to provide metal-chelating complexes that are nonionic.

The invention also provides the possibility of providing metal chelating ligands (a) which when complexed with a metal heavier than iodine (e.g. Ba, Ta, Pb, Bi, Lanthanides) are effective as X-ray contrast agents, (b) which when complexed with gamma emitting radioactive nuclide (e.g. $^{99m}$Tc or $^{111}$In) are effective as imaging radiopharmaceuticals, and/or (c) which when complexed with beta or alpha emitting radioactive nuclide (e.g. $^{90}$Y, $^{153}$Sm, $^{188}$Re, $^{212}$Bi) are effective as therapeutic radiopharmaceuticals.

Using the invention, it is possible to provide metal-chelating ligands whose metal chelate complexes in aqueous solution have low osmolality and whose metal chelate complexes have low acute toxicity, and which, when complexed with a paramagnetic metal atom, are effective as relaxation catalysts in magnetic

resonance imaging.

It is furthermore possible to provide bifunctional metal-chelating ligands that have the ability to covalently bind to proteins or other biologically active molecules thereby imparting biological specificity to the metal chelate complex, and bifunctional metal-chelating ligands that are then modynamically stable, kinetically inert and, when desired, electrically neutral.

The present invention provides compounds having the formula

I.

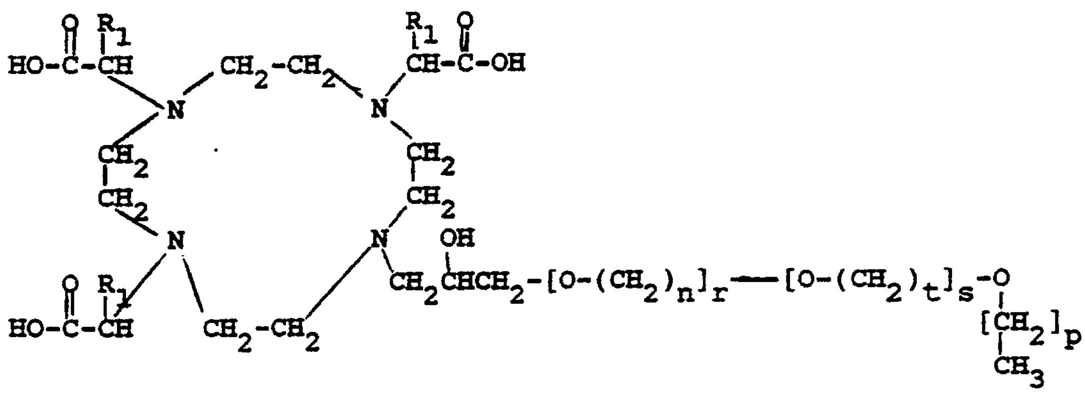

wherein

n is an integer from two to five;

p is zero or one; r is an integer from one to five; S is zero or an integer from one to five and t is an integer from two to five. Prefered values are n = two or three; r = one, two or three, t = two or three and S = zero, one or two and p is zero or one. Most preferred is n = 2, r = 1, t = 2, s = 1 and p = 1.

$R_1$ is hydrogen or alkyl.

The term "alkyl" as used throughout the specification, refers to both straight and branched chain groups. Those groups having 1 to 5 carbon atoms are preferred and methyl is the most preferred alkyl group.

The compounds of formula I, and salts thereof, can be complexed with a paramagnetic metal atom and used as relaxation enhancement agents for magnetic resonance imaging. These agents, when administered to a mammalian host (e.g., humans) distribute in various concentrations to different tissues, and catalyze relaxation of protons (in the tissues) that have been excited by the absorption of radio-frequency energy from a magnetic resonance imager. This acceleration of the rate of relaxation of the excited protons provides for an image of different contrast when the host is scanned with a magnetic resonance imager. The magnetic resonance imager is used to record images at various times generally before and after administration of the agents, and the differences in the images created by the agents' presence in tissues are used in diagnosis. In proton magnetic resonance imaging, paramagnetic metal atoms such as gadolinium(III), and octahedral manganese(II), chromium(III), and iron(III) (all are paramagnetic metal atoms with a symmetrical electronic configuration) are preferred as metals complexed by the ligands of formula I; gadolinium (III) is most preferred due to the fact that it has the highest paramagnetism, low toxicity, and high lability of coordinated water.

The metal-chelating ligands of formula I can be complexed with a lanthanide (atomic number 58 to 71) and used as chemical shift agents in magnetic resonance imaging or in magnetic resonance *in vivo* spectroscopy.

While the above-described uses for the metal-chelating ligands of formula I are preferred, those working in the diagnostic arts will appreciate that the ligands can also be complexed with the appropriate metals and used as contrast agents in x-ray imaging, radionuclide imaging and ultrasound imaging.

## Use In Imaging

To use the ligands of this invention for imaging, they must first be complexed with the appropriate metal. This can be accomplished by methodology known in the art. For example, the metal can be added to water in the form of an oxide or in the form of a halide and treated with an equimolar amount of a ligand of formula I. The ligand can be added as an aqueous solution or suspension. Dilute acid or base can be added

(if needed) to maintain a neutral pH. Heating at temperatures as high as 100° C for periods up to four hours is sometimes required, depending on the metal and the chelator, and their concentrations.

Pharmaceutically acceptable salts of the metal complexes of the ligands of this invention are also useful as imaging agents. They can be prepared by using a base (e.g., an alkali metal hydroxide, meglumine or arginine) to neutralize the above-prepared metal complexes while they are still in solution. Some of the metal complexes are formally uncharged and do not need cations as counterions. Such neutral complexes are preferred as intravenously administered x-ray and NMR imaging agents over charged complexes because they provide solutions of greater physiologic tolerance due to their lower osmolality.

Sterile aqueous solutions of the chelate-complexes can be administered to mammals (e.g., humans) orally, intrathecally and especially intravenously in concentrations of 0.003 to 1.0 molar. For example, for the visualization of brain lesions in canines using magnetic resonance imaging, a gadolinium complex of a ligand of formula I can be administered intravenously at a dose of 0.05 to 0.5 millimoles of the complex per kilogram of animal body weight, preferably at a dose of 0.1 to 0.25 millimole/kilogram. For visualization of the kidneys, the dose is preferably 0.05 to 0.25 millimoles/kilogram. For visualization of the heart, the dose is preferably 0.25 to 1.0 millimoles/kilogram. The pH of the formulation will be between about 6.0 and 8.0, preferably between about 6.5 and 7.5. Physiologically acceptable buffers (e.g., tris(hydroxymethyl)-aminomethane) and other physiologically acceptable additives (e.g., stabilizers such as parabens) can be present.

The compounds of formula I can be prepared by following the reaction scheme described below:

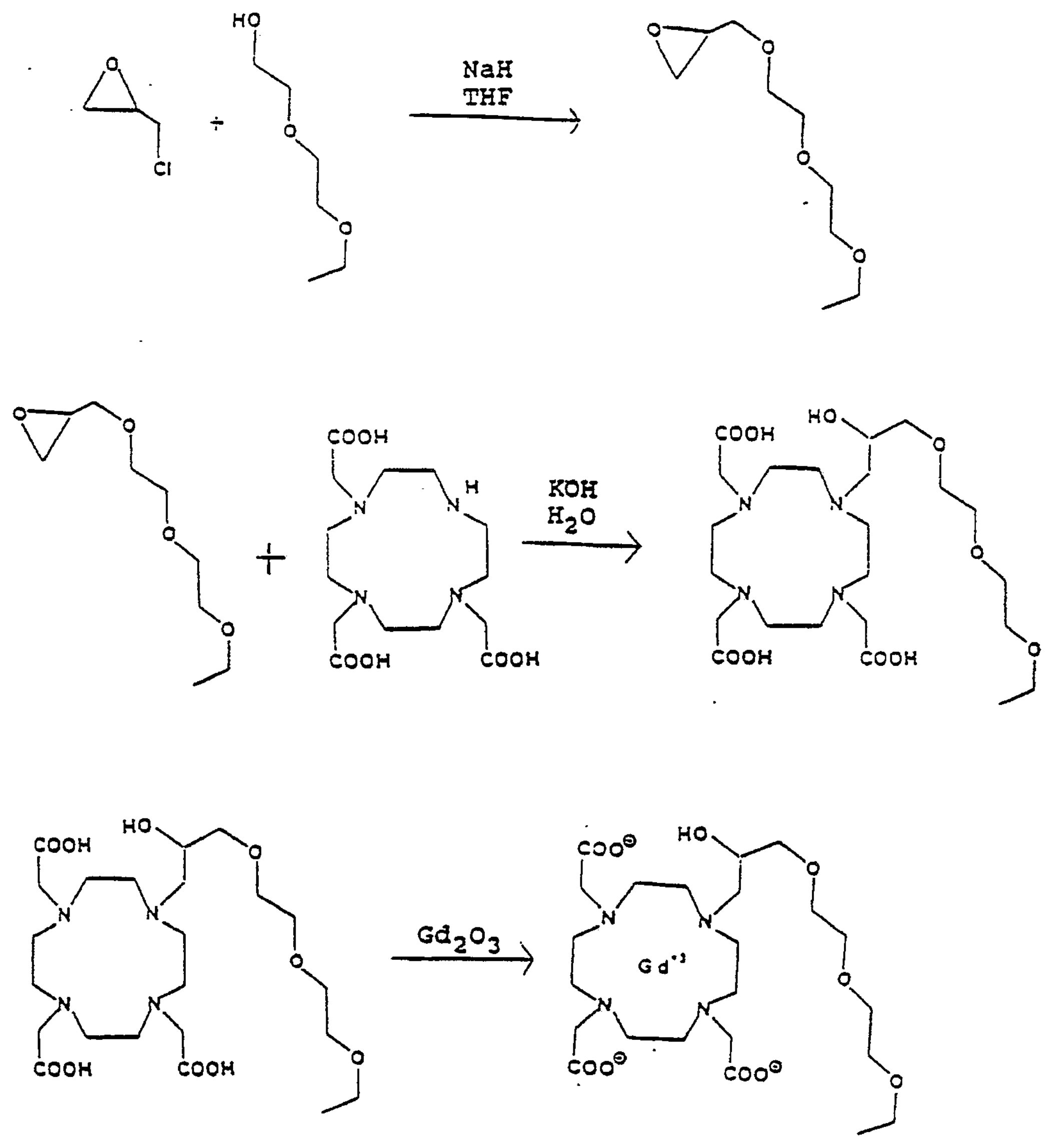

The various compounds of formula 1 are made by varying the starting polyether alcohol.

The following examples are specific embodiments of this invention.

Example 1

a) 1,2-Epoxy-4,7,10-trioxadodecane

NaH (4.45 g, 0.111 mole of 60% dispersion in oil) was rinsed with 10 ml dry pentane to remove the oil. The NaH was suspended in 100 ml dry THF under $N_2$ and to this was added freshly distilled ethoxy-ethoxyethanol (12.45 g, 0.0928 mole). The resulting suspension was stirred 2 hours at ambient temperature. Epichlorohydrin (25.40 ml, 0.325 mole) was added dropwise and stirring was continued overnight. The reaction was heated to reflux 4 hours, then cooled and filtered to remove precipitated salts. The filtrate was dried over $MgSO_4$, filtered, and evaporated to an oily residue. The product was isolated by distillation (b.p. 85 - 90° C, 1 mm Hg) as a clear oil (14.07 g., 80% yield).

b) 1,4,7-tris(carboxymethyl)-10-(2'-hydroxy-4',7',10'-trioxadodecyl)-1,4,7,10-tetraazacyclododecane

A solution of 13.84 g. (0.040 mole, corrected for 2.5% $H_2O$ of 1,4,7-tris(carboxymethyl)-1,4,7,10-tetraazacyclododecane in 200 ml of $H_2O$ was adjusted to pH 10.5 using 5M KOH. To this was added 8.37 g. (0.44 mole) of 1,2-epoxy-4,7,10-trioxadodecane. The resulting cloudy solution was stirred at ambient temperature and the pH was kept at 10 - 10.5 by occasional additions of KOH.

After four days, the reaction mixture was applied to a 10 x 21 cm column of AG1 - X8 anion exchange resin, formate form. The column was eluted with 8 L of $H_2O$ at a flow rate of 11 ml/min. The column was then eluted with 3 L of 0.5 M $HCO_2H$, and the eluate collected and evaporated to a glassy residue. The residue was dissolved in 200 ml. of $H_2O$ and applied to a 4.5 x 40 cm column of previously regenerated poly-4-vinylpyridine. The column was eluted with 3 L $H_2O$. The $H_2O$ was evaporated and the residue was dried under vacuum at ambient temperature for 18 hours. The yield was 18.1 g (82% corrected for 2.45% $H_2O$) of 1,4,7-tris(carboxymethyl)-10-(2'-hydroxy-4',7',10'-trioxadodecyl)-1,4,7,10-tetraazacyclododecane.
Anal. Calcd (found) for $C_{23}H_{44}N_4O_{10}\cdot 0.75H_2O$:
C, 50.21 (50.26); H, 8.34 (8.51); N, 10.19 (10.53) $H_2O$, (2.45 by dissolution KF)

Example 2

1,4,7-tris(carboxymethyl)-10-(2'-hydroxy-4',7',10'-trioxadodecyl)-1,4,7,10-tetraazacyclododecanato-gadolinium

1,4,7-tris(carboxymethyl)-10-(2'-hydroxy-4',7',10'-trioxadodecyl)-1,4,7,10-tetraazacyclododecane (13.4 g, 0.0244 mole corrected for 2.45% $H_2O$) was dissolved in 250 ml $H_2O$ to give a solution of pH 3.4. $Gd_2O_3$ - (4.76 g, 0.0131 mole) was added and the suspension was stirred at 80° C. for 18 hours. The resulting cloudy solution was cooled to ambient temperature and filtered through a 0.2 micron membrane. The solution was adjusted to pH 9.4 using conc. $NH_4OH$, and applied to a 10 X 20 cm column of Chelex 100, ammonium form. The column was eluted with 2.5 L $H_2O$. The eluate collected and evaporated to yield a white glassy solid.

The chelate was further purified by preparative reverse-phase HPLC. The fractions containing the chelate were combined and evaporated to give a white solid.

The solid was twice crystallized from hot 95% EtOH. A second crop was obtained when the mother liquors were evaporated and the residue crystallized as above. The two crops were combined and dried under high vacuum overnight at 58° C., to give 7.13 g. (42% corrected for 2.26% $H_2O$) of 1,4,7-tris-(carboxymethyl)-10-(2'-hydroxy-4',7',10'-trioxadodecyl)-1,4,7,10-tetraazacyclododecanato-gadolinium.
Anal Calcd. (Found) for $C_{23}H_{41}N_4O_{10}$ Gd·0.89 $H_2O$:
C, 39.08(39.11); H, 6.10 (6.33); N, 7.93 (7.96); $H_2O$, (2.26 by dissolution KF).

## Claims

1. A compound having the formula

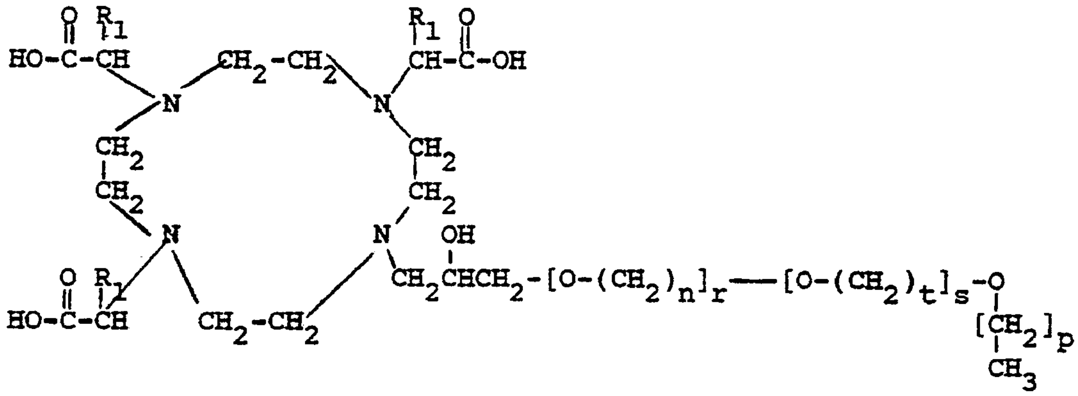

wherein n is an integer from two to five, r is an integer from one to five, t is an integer from two to five, s is zero or an integer from one to five and p is zero or one,
$R_1$ is hydrogen or alkyl.

2. A compound according to Claim 1 wherein n is two or three.

3. A compound according to Claim 1 or 2 wherein r is one, two or three.

4. A compound according to any preceding claim wherein t is two or three.

5. A compound according to any preceding claim wherein s is zero, one or two.

6. A compound acccrding to any preceding claim wherein p is zero or one.

7. A compound according to claim 1 wherein n is two, r is one, t is two, s is one and p is one.

8. A compound according to claim 1, 1,4,7-tris-(carboxymethyl)-10-(2'-hydroxy-4',7',10'-trioxadodecyl)-1,4,7,10-tetraazacyclododecane.

9. A complex, or a salt of a complex, of a metal atom and a metal-chelating ligand having the formula

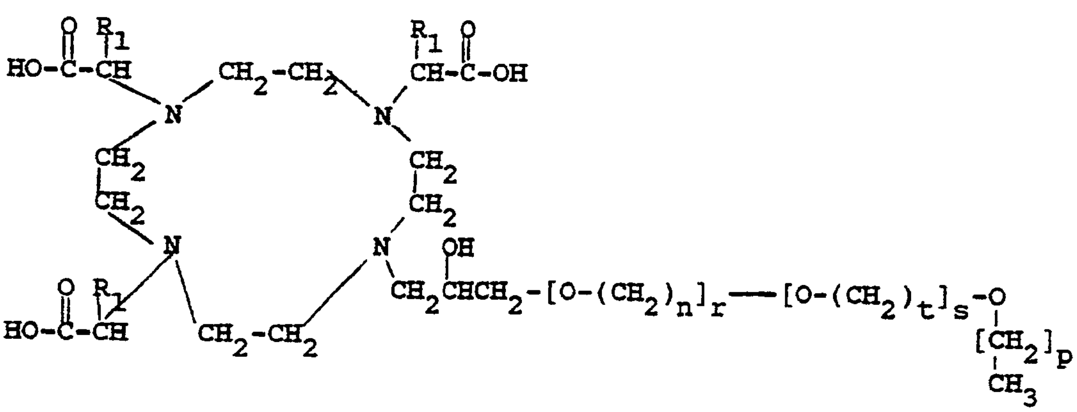

wherein n is an integer from two to five, r is an integer from one to five, t is an integer from two to five, s is zero or an integer from one to five and p is zero or one,
$R_1$ is hydrogen or alkyl.

10. A complex according to claim 9 where the metal atom is gadolinium (III).

**11.** A complex in accordance with Claim 9, 1,4,7-tris-(carboxymethyl)-10-(2'-hydroxy-4',7',10'-trioxadodecyl)-1,4,7,10-tetraazacyclododecanato-gadolinium.

**12.** A process for preparing compounds having the formula

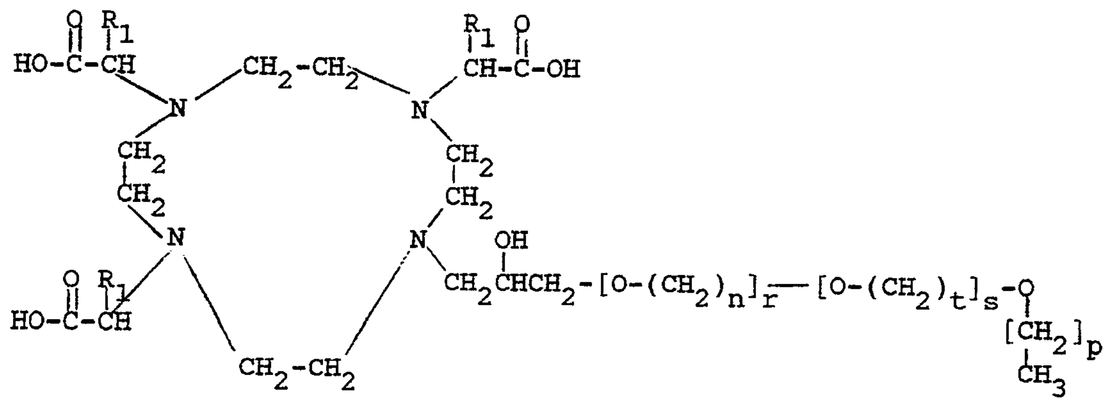

n is an integer from two to five;
p is zero or one; r is an integer from one to five; s is zero or an integer from one to five and t is an integer from two to five by reacting a compound having the formula

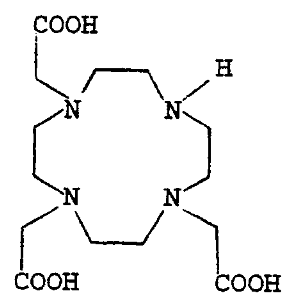

with a compound of the formula

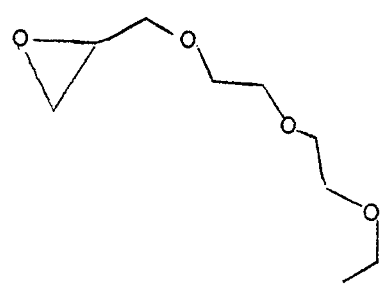

in the presence of a strong base and water.

**13.** A process according to Claim 12 wherein the resulting ligand is reacted with a metal oxide in water to form the corresponding metal complex.

**14.** A process according to Claim 13 wherein the metal oxide is $Gd_2O_3$.

**15.** A complex according to any one of Claims 9-11 for use as a diagnostic contrast agent.

European Patent Office

# EUROPEAN SEARCH REPORT

Application Number

EP 90313791.7

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | DE - A1 - 3 625 417 (SCHERING) * Claims 1,6,8; example 6 * -- | 1,9, 10,15 | C 07 D 257/02 A 61 K 49/04 |
| A | EP - A2 - 0 292 689 (SQUIBB) * Claims 1,62 * -- | 1,9, 15,10, 11 | |
| A | EP - A1 - 0 232 751 (SQUIBB) * Claims 1,17 * -- | 1,9, 10,15, 11 | |
| A | EP - A1 - 0 287 465 (GUERBET) * Claims 1,16 * -- | 1,10, 15,11 | |
| A | EP - A1 - 0 326 226 (NYCOMED) * Claims 1,23 * -- | 1,15 | |
| A | EP - A1 - 0 299 795 (NYCOMED) * Claims 7,12 * ---- | 1,15 | TECHNICAL FIELDS SEARCHED (Int. Cl.5) C 07 D 257/00 |

The present search report has been drawn up for all claims

| Place of search | Date of completion of the search | Examiner |
|---|---|---|
| VIENNA | 01-03-1991 | HAMMER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)